# EUROPEAN PATENT SPECIFICATION

(11) **EP 2 424 470 B1**
(45) Date of publication and mention of the grant of the patent: **07.08.2013**
(21) Application number: 10719158.7
(22) Date of filing: 28.04.2010
(51) Int. Cl.: A61F 2/966, A61F 2/95

(54) **INTRODUCER ASSEMBLY AND METHOD OF MANUFACTURING AN INTRODUCER ASSEMBLY**
EINFÜHRUNGSANORDNUNG UND VERFAHREN ZUR HERSTELLUNG EINER EINFÜHRUNGSANORDNUNG
ENSEMBLE INTRODUCTEUR ET PROCÉDÉ DE FABRICATION D'UN ENSEMBLE INTRODUCTEUR

(30) Priority: 28.04.2009 GB 0907291
(43) Date of publication of application: 07.03.2012
(73) Proprietor: Cook Medical Technologies LLC, Bloomington, IN 47404 (US)
(72) Inventor: RASMUSSEN, Erik, Edelboe, DK-4200 Slagelse (DK); OEHLENSCHLAEGER, Bent, DK-4623 Li. Skensved (DK); PETERSEN, Jesper, Schade, DK-4684 Holmegaard (DK)
(74) Representative: Georgiou, Sarah Caroline
(86) International application number: PCT/US2010/032704
(87) International publication number: WO 2010/126964

(56) References cited:
- WO-A1-00/48517
- US-A1- 2008 172 122
- US-A1- 2009 088 771

## Description

### Technical Field

The present invention relates to an introducer assembly, for example for introducing stents, stent grafts or other implantable medical devices into a patient, and to a method of making such an introducer assembly.

### Background Art

Procedures for carrying out endoluminal treatments on a patient are well known in the art and have been practiced for a number of decades. Such procedures can involve the treatment of a diseased or damaged vessel or organ but they also involve the introduction and implantation of a medical device, such as a stent, stent graft, vena cava filter and so on. For example, procedures can be carried out in the aortic region, all the way up to and beyond the aortic arch. In procedures of this nature, it is common to pass through a patient's femoral artery up to the aortic region. The femoral artery is readily accessible for such procedures.

It is typical for introducers and other deployment devices of this nature to have a significant length, lengths of 1.5m not being uncommon. These introducers must be flexible so as to have adequate trackability within the vasculature of a patient, that is they must be able to follow the tortuous path of the vessels up to and sometimes beyond the zone where treatment is required without being so stiff as to cause damage to the walls of the patient's vasculature. This flexibility, given in particular the length of such introducers, causes problems with twisting of the introducer during its use, particularly as a surgeon or other clinician works to push and guide the introducer through the vasculature of a patient (typically over a pre-inserted guide wire). The introducer will normally twist along its length as a result of the torque applied to it as it is being inserted into the patient.

Introducer assemblies of this nature, particularly introducers intended to deploy an implantable medical device, are typically made of a plurality of components located concentrically one within the other. For example, a typical introducer for introducing a stent or stent graft into a patient will have a cannula or catheter through which a guide wire passes, a pusher or similar element for urging the implantable medical device through the vasculature of the patient and a cover, typically an outer sheath, protecting the internal components. The skilled person will appreciate that there are many other components to such introducer assemblies.

As a result of this multi-part structure of introducers, when the introducer is rotated, for example twisted during its passage through a patient, there is typically a difference in the torque applied to the various elements of the assembly, with the result that these can rotate or twist relative to one another, particularly at a distal end of the introducer remote from the external manipulation end used and handled by the surgeon or other clinician during the deployment procedure.

If the components of the introducer twist relative to one another, particularly at the deployment end where the implantable medical device is located, there is a chance that such twisting will cause incorrect deployment of the implantable medical device (for example it may be incorrectly rotated). This can also damage the implantable medical device due to its being located between components which are twisting relative to one another. Any excessive twisting of this nature can result in the introducer and implantable medical device being unusable and potentially result not only in wastage of the introducer and medical device but also in an aborted medical procedure.

Various designs of the introducer assemblies are disclosed in US-2007/0208407, WO-2005/030093, and US-5,201 ,757.

US2008/0172122 discloses a guidewire assembly for use in deploying a bifurcated endoluminal vascular prosthesis that has a main graft portion and at least a first branch graft portion. The guidewire assembly includes a hollow guidewire sheath having a restraint mechanism, such as a tubular sheath, for constraining a branch graft portion of the vascular prosthesis and an inner core wire that is slidably insertable into a central lumen of the hollow guidewire sheath. US2008/0172122 mentions rotationally locking together particular tubes of the deployment apparatus.

### Disclosure Of The Invention

The present invention seeks to provide an improved introducer assembly and an improved method of making an introducer assembly as disclosed in the appended claims.

According to an aspect of the present invention as disclosed in claim 1, there is provided an introducer assembly for deploying an implantable medical device, the assembly including a guide wire catheter, a pusher element located on the catheter and an external manipulation unit; the guide wire catheter and pusher element including proximal and distal ends, the proximal ends of the guide wire catheter and the pusher element being connected to the external manipulation unit; wherein the pusher element is rotationally fixed to the guide wire catheter at the distal end of the pusher element by means of a rotation prevention device.

Typically, in prior art systems which include a pusher element and a guide wire catheter, the pusher element is only fixed relative to the guide wire catheter at the proximal end of the introducer element but otherwise they are not connected to one another.

The rotation prevention device includes a bonding element located at or proximate the distal end of the pusher element.

The application of a bonding element fixes the distal end of the pusher element to the guide wire catheter. In some embodiments, the bonding element fixes the distal end of the pusher element permanently to the guide wire catheter so that there can be no relative movement between the two at the point of bonding. In other embodiments, the bonding element could be rupturable to allow relative movement between the guide wire catheter and the pusher element, for example to allow the pusher element to move in a longitudinal direction relative to the guide wire catheter in cases where this is desirable.

Bonding the distal end of the pusher element in this manner ensures that any twisting of the introducer assembly from the external manipulation end will provide equivalent twisting of the guide wire catheter and the pusher element to the position of the bonding and therefore will substantially reduce or prevent any relative rotation between these two elements. This will thus substantially reduce any adverse effects on the implantable medical device carried on the introducer caused by it being twisted during the deployment operation. In the example of an introducer for deploying an aortic medical device, in prior art systems the effect of any twist of the introducer element at the external manipulation end would be carried for the full length of the introducer assembly, that is potentially well over one meter. This can result in a significant difference in rotation between the guide wire catheter and the pusher element as a result of differing rotational stiffnesses of these two components. However, with the provision of bonding agent, any twist at the external manipulation end will be transmitted along the length of both the guide wire catheter and the pusher element and thus the distance over which the two are not fixed together in rotation is reduced to a few tens of centimeters at most and typically no more than around 10 to 15 cm. As a result of this, any residual twisting between the guide wire catheter and the pusher element appears only from the position of the bonding agent and therefore is very substantially reduced. This can substantially avoid twisting of the medical device carried on the introducer and can thus provide more accurate deployment of the medical device and reduced instance of damage to the device during the deployment operation.

The bonding agent may be a glue, such as ciano acrylate, an ultraviolet curable glue. In some instances, the bonding agent could also be a melted portion of the pusher element and/or guide wire and it is envisaged it could also be provided by a mechanical coupling.

It will be appreciated also that in bonding the pusher element to the guide wire catheter, there is produced a stronger assembly which is able to transfer additional torque to the distal end of the introducer assembly, thereby facilitating the process of orienting implantable medical device within a lumen of a patient, particularly once this has already been located at the treatment site.

According to another aspect of the present invention as disclosed in claim 7, there is provided a method of making an introducer assembly including providing a guide wire catheter, fitting concentrically over the guide wire catheter an elongate pusher element; the guide wire catheter and pusher element having distal ends and proximal ends; fitting to the proximal ends of the pusher element and guide wire catheter an external manipulation unit; and providing a rotation prevention device at or proximate the distal end of the pusher element between the pusher element and the guide wire catheter so as to bond the distal end of the pusher element to the guide wire catheter.

### Brief Description of the Drawings

The embodiments of the present invention are described below, with reference to the accompanying drawings, in which:
Figure 1 shows an example of the major components of an introducer assembly; and
Figure 2 shows a preferred embodiment of an introducer assembly.

### Description of the Preferred Embodiments

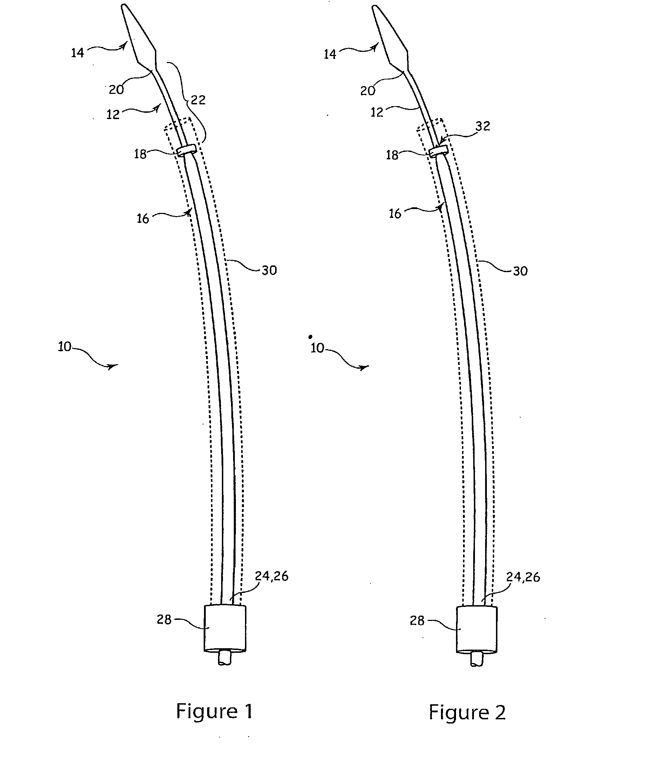

Referring to Figure 1, there are shown the major components of an example of introducer assembly 10 of a type suitable for deploying stents, stent grafts, vena cava filters, occlusion devices and the like within the vasculature or an organ of a patient. The introducer assembly includes a guide wire catheter 12, which is typically formed as a narrow flexible cannula with a lumen therethrough for receiving a guide wire (not shown but well known in the art). At a distal end of the guide wire catheter there is provided a dilator tip 14, also of known form. Disposed over the guide wire catheter 12 is a pusher element 16 which extends for a substantial length of the guide wire catheter 12. The pusher element 16 ends, at its distal end 18, short of the distal end 20 of the guide wire catheter 12, so as to leave a zone 22 which forms the medical device carrier portion of the introducer assembly. In other words, it is in the zone 22 that the medical device to be implanted in a patient is located.

In this example, the distal end 18 of the pusher element is provided with what could be described as an enlarged abutment shoulder against which an implantable medical device is positioned. This shoulder allows the pusher element 16 to be used in pushing the implantable medical device through the vasculature of the patient and during the process of deploying the medical device from the introducer 10.

The proximal ends 24, 26 of the guide wire catheter 12 and pusher element 16 are connected to an external manipulation unit 28, of conventional form. The external manipulation unit 28 typically includes a handle element or feature grippable by the clinician, one or more valves for sealing the introducer assembly 10, one or more fluid ports for supplying and/or flushing through the introducer assembly 10 and so on. The components of such an external manipulation unit 28 are well known to the person skilled in the art and are therefore not described in further detail herein.

The introducer assembly 10 is also provided with an external sheath 30 which extends along substantially the entirety of the length of the assembly 10 and in use envelopes the guide wire catheter 12, the pusher element 16 and an implantable medical device carried on the introducer. The sheath typically extends to the distal end of the guide wire catheter 12 and proximal end of the dilator tip 14.

The outer sheath 30 is movable relative to the other elements of the introducer assembly so as to be able to be withdrawn backwards from the distal end 20 of the guide wire catheter 12 in a direction towards the proximal end of the introducer assembly 10, so as to uncover gradually the implantable medical device carried on the guide wire catheter 20 at the zone 22. Sheaths of this nature are well known in the art.

In the example of Figure 1, the guide wire catheter 12 and the pusher element 16 are in effect coupled to one another through their connections to and in the external manipulation unit 28. They are not connected to one another at any other point along their lengths. As a result of this, should the external manipulation unit 28 be twisted or rotated during the introduction of the introducer assembly 10 into the vasculature of a patient, which is typically the case, the amount by which the distal ends 18, 20 of the pusher element 16 and guide wire catheter 12 will rotate will be determined at least in part by the relative rigidities in rotation (torque resistance) of these elements individually. As these rotational rigidities are not generally matched, there will be a tendency at least for the distal ends 18, 20 to rotate by different amounts when the external manipulation unit 28 is twisted. This can cause potentially significant twisting of a medical device carried on the introducer assembly.

Referring now to Figure 2, there is shown a preferred embodiment of the present invention. In Figure 2, the same components of the introducer 10 are shown as in the example of Figure 1.

In this embodiment, there is provided at the distal end 18 of the pusher element a bonding agent 32 between the pusher element 16 and the guide wire catheter 12 so as to bond the distal end 18 of the pusher element to the guide wire catheter 12. Any suitable glue could be used as a bonding agent but it is preferred an ultraviolet curable glue be used. A preferred glue is ciano acrylate. It is not, however, excluded that in some embodiments the distal end 18 of the pusher rod or pusher element 16 could be welded to the guide wire catheter 12, for example by applying heat to this so as to cause this to melt onto the guide wire catheter and provide the desired bond.

In many instances, the bonding of the distal end 18 of the pusher element 16 to the guide wire catheter 12 can be an effective permanent bond, that is a bond which will not release during normal operation of the introducer assembly 10. It is envisaged, however, that in some embodiments that bond could be breakable during the deployment process, for example in cases where it is desired to have relative movement of the guide wire catheter 12 and the pusher element 16 during the deployment of an implantable medical device carried on the introducer. In this case, the bonding agent used could be breakable (for example by applying pressure in the longitudinal direction of the introducer assembly through the guide wire catheter or the pusher element 16) or by use of a smaller amount of bonding agent, thereby to create a weaker bond. In the event of a breakable bond of this nature, it is, of course, envisaged that this would break upon a specific action by the clinician and specific operation of the introducer assembly 12 rather than by simple twisting of the assembly during the deployment process.

Having the distal end 18 of the pusher element 16 bonded to the guide wire catheter 12 provides a number of advantages. First, the bond 32 ensures that any twisting of the introducer assembly 10 during the deployment operation will be mirrored in both of the pusher element 16 and the guide wire catheter up to at least the point of the bond 32. This reduces very substantially the amount of relative twisting between these two components, particularly in cases where the pusher element 16 is around 1 meter or more in length and the section 22 of the guide wire catheter 12 extending beyond the distal end 18 of the pusher element 16 is only 10 or 15 cm or so in length. Thus, there is a substantially reduced instance of twisting of the medical device carried on the introducer assembly and therefore a substantially reduced chance of damage to the implantable medical device.

Furthermore, by reduction of such twisting, the medical device can be deployed more precisely at the target site within a patient.

In addition to these advantages, bonding these two components 12 and 16 together, will increase their torque rigidity, thereby reducing the overall twisting of the distal end of the introducer assembly. This therefore provides an introducer assembly with increased torque resistance compared to prior art devices.

In some embodiments it may be desirable to construct the pusher element 16 to have a substantially greater torque resistance than conventional pusher elements, possible by the fact that it is bonded at its distal end to the guide wire catheter. An embodiment of higher torque resistance pusher element 16 is formed with a braided element, provided for example by a metal or metallic wire braid. Given the fact that the pusher element 16 and guide wire catheter 12 are bonded to one another in the manner described above, it is considered that there is no disadvantage in having substantially different torque resistances between these two components as the torque resistance of one component (in this case the pusher element 16) will contribute to the torque resistance of the other component (in this case the guide wire catheter 12). In other words, the two elements will rotate as one in the introducer assembly.

In some examples it is envisaged that bonding agent 32 could be applied at other locations along the pusher element 16, and in particular also at the proximal end 26, so as to bond this end 26 to the proximal end 24 of the guide wire catheter 12.

It is also envisaged in another example that the distal end 18 of the pusher element 16 could be rotationally fixed to guide wire catheter 12 by a mechanical coupling rather than by bonding as with the above-described embodiments. This may, for instance, be by providing a rotation fixing arrangement at the distal end 18 of the pusher element 16 and at the corresponding position of the guide wire catheter 12 which is non-round. In one specific example, the internal surface of the end 18 of the pusher element 16 could be modified, by machining for example, to have a polygonal shape, with a corresponding shape being produced in the same longitudinal position on the outside of the wide guide catheter 12. A polygonal shape would rotationally lock the distal end 18 of the pusher element 16 to the wire guide catheter 12 and thus have the same effect and the bonding element 32 described above. It is also envisaged that there could be provided a non-round configuration at the distal end of the outer sheath 30, made to cooperate with a corresponding non-round shape at the proximal end 20 of the dilator tip 14.

## Claims

1. An introducer assembly (10) for deploying an implantable medical device, the assembly including a guide wire catheter (12), a pusher element (16) located on the catheter (12) and an external manipulation unit (28); the guide wire catheter (12) and pusher element (16) including proximal (24, 28) and distal ends (20, 18), the proximal ends (24, 26) of the guide wire catheter (12) and the pusher element (16) being connected to the external manipulation unit (28); wherein the pusher element (16) is rotatianally fixed to the guide wire catheter (12) at the distal end (18) of the pusher element (16) by means of a rotation prevention device; and **characterized in that**: the rotation prevention device includes a bonding element (32) located at or proximate the distal end of the pusher element.

2. An assembly according to claim 1, wherein the bonding element (32) provides an effective permanent connection between the distal end of the pusher element and the guide wire catheter.

3. An assembly according to claim 1. wherein the bonding element (32) is rupturable to allow relative movement between the guide wire catheter and the pusher element.

4. An assembly according to claim 1, wherein the bonding element (32) is an ultraviolet curable glue.

5. An assembly according to claim 1, wherein the bonding element (32) is a ciano acrylate glue.

6. An assembly according to claim 1, wherein the bonding element (32), is a melted portion of one of the pusher element and the guide wire catheter.

7. A method of making an introducer assembly (10) including the steps of:
providing a guide wire catheter (12);
fitting concentrically over the guide wire catheter (12) an elongate pusher element (16), the guide wire catheter (12) and pusher element (16) having distal ends (20, 18) and proximal ends (24, 26);
fitting to the proximal ends (24, 26) of the pusher element (16) and guide wire catheter (12) an external manipulation unit (28); and
providing a rotation prevention device at or proximate the distal end (18) of the pusher element (16) between the pusher element (16) and the guide wire catheter (12) so as to bond the distal end (18) of the pusher element (16) to the guide wire catheter (12); wherein the step of providing a rotation prevention device includes the step of applying a bonding element (32) at or proximate the distal end (18) of the pusher element (16) between the pusher element (16) and the guide wire catheter (12).

8. A method according to claim 7, wherein the bonding element (32) provides an effective permanent connection between the distal end (18) of the pusher element (16) and the guide wire catheter (12).

9. A method according to claim 7, wherein the bonding element (32) is rupturable to allow relative movement between the guide wire catheter (12) and the pusher element (16).

10. A method according to claim 7, wherein the bonding element (32) Is an ultraviolet curable glue.

11. A method according to claim 7, wherein the bonding element (32) is a ciano acrylate glue.

12. A method according to claim 7, wherein the bonding element (32), is a melted portion of the pusher element (16) and/or guide wire catheter (12) and the method includes the step of melting a portion of the pusher element (16) and/or guide wire catheter (12) to provide said bonding element (32).

## Patentansprüche

1. Einführungsanordnung (10) zum Einsetzen einer implantierbaren medizinischen Vorrichtung, wobei die Anordnung einen Führungsdrahtkatheter (12), ein Schiebeelement (16), welches an dem Katheter (12) positioniert ist, und eine außenliegende Manipulationseinheit (28) aufweist; der Führungsdrahtkatheter (12) und das Schiebeelement (16) proximale (24, 26) und distale Enden (20, 18) aufweisen, wobei die proximalen Enden (24, 26) von dem Führungsdrahtkatheter (12) und dem Schiebeelement (16) mit der außenliegenden Manipulationseinheit (28) verbunden sind; wobei das Schiebeelement (16) drehfest mit dem Führungsdrahtkatheter (12) an dem distalen Ende (18) des Schiebeelements (16) mittels einer Verdrehsicherungs-Vorrichtung verbunden ist; und **dadurch gekennzeichnet ist, dass**: die Verdrehsicherungs-Vorrichtung ein Verbindungselement (32) aufweist, das an oder in der Nähe von dem distalen Ende des Schiebelements positioniert ist.

2. Anordnung gemäß Anspruch 1, wobei das Verbindungselement (32) eine wirkungsvolle dauerhafte Verbindung zwischen dem distalen Ende von dem Schiebelement und dem Führungsdrahtkatheter bereitstellt.

3. Anordnung gemäß Anspruch 1, wobei das Verbindungselement (32) aufreißbar ist, um eine relative Bewegung zwischen dem Führungsdrahtkatheter und dem Schiebeelement zu ermöglichen.

4. Anordnung gemäß Anspruch 1, wobei das Verbindungselement (32) ein durch UV-Strahlung härtbarer Kleber ist.

5. Anordnung gemäß Anspruch 1, wobei das Verbindungselement (32) ein Cyanacrylatkleber ist.

6. Anordnung gemäß Anspruch 1, wobei das Verbindungselement (32) ein geschmolzener Abschnitt von dem Schiebeelement oder dem Führungsdrahtkatheter ist.

7. Verfahren zur Herstellung einer Einführungsanordnung (10), welches die folgenden Schritte aufweist:
Vorsehen eines Führungsdrahtkatheters (12);
Montieren eines langgestreckten Schiebeelements (16) konzentrisch über den Führungsdrahtkatheter (12), wobei der Führungsdrahtkatheter (12) und das Schiebeelement (16) distale Enden (20, 18) und proximale Enden (24, 26) aufweisen;
Montieren einer außenliegenden Manipulationseinheit (28) an die proximalen Enden (24, 26) des Schiebeelements (16) und des Führungsdrahtkatheters (12); und
Vorsehen einer Verdrehsicherungs-Vorrichtung am oder in der Nähe des distalen Endes (18) des Schiebeelements (16) zwischen dem Schiebeelement (16) und dem Führungsdrahtkatheter (12) derart, dass das distale Ende (18) von dem Schiebeelement (16) an den Führungsdrahtkatheter (12) gebunden wird; wobei der Schritt des Vorsehens einer Verdrehsicherungs-Vorrichtung den Schritt des Anbringens eines Verbindungselements (32) an oder in der Nähe von dem distalen Ende (18) des Schiebeelements (16) zwischen dem Schiebeelement (16) und dem Führungsdrahtkatheter (12) umfasst.

8. Verfahren gemäß Anspruch 7, wobei das Verbindungselement (32) eine wirkungsvolle dauerhafte Verbindung zwischen dem distalen Ende (18) des Schiebeelements (16) und dem Führungsdrahtkatheter (12) vorsieht.

9. Verfahren gemäß Anspruch 7, wobei das Verbindungselement (32) aufreißbar ist, um eine relative Bewegung zwischen dem Führungsdrahtkatheter (12) und dem Schiebeelement (16) zu ermöglichen.

10. Verfahren gemäß Anspruch 7, wobei das Verbindungselement (32) ein durch UV-Strahlung härtbarer Kleber ist.

11. Verfahren gemäß Anspruch 7, wobei das Verbindungselement (32) ein Cyanacrylatkleber ist.

12. Verfahren gemäß Anspruch 7, wobei das Verbindungselement (32) ein geschmolzener Abschnitt von dem Schiebeelement (16) und/oder dem Führungsdrahtkatheter (12) ist und das Verfahren den Schritt des Schmelzens eines Abschnitts von dem Schiebeelement (16) und/oder dem Führungsdrahtkatheter (12) umfasst, um das Verbindungselement (32) vorzusehen.

## Revendications

1. Ensemble d'introduction (10) servant à déployer un dispositif médical implantable, l'ensemble comprenant un cathéter (12) à fil de guidage, un élément poussoir (16) situé sur le cathéter (12) et une unité externe de manipulation (28),
le cathéter (12) à fil de guidage et l'élément poussoir (16) présentant des extrémités proximales respectives (24, 26) et des extrémités distales respectives (20, 18), les extrémités proximales (24, 26) du cathéter (12) à fil de guidage et de l'élément poussoir (16) étant raccordées à l'unité externe de manipulation (28),
l'élément poussoir (16) étant fixé à rotation sur le cathéter (12) à fil de guidage à l'extrémité distale (18) de l'élément poussoir (16) au moyen d'un dispositif de prévention de la rotation, **caractérisé en ce que**
le dispositif de prévention comprend un élément de liaison (32) situé à l'extrémité distale de l'élément poussoir ou à proximité de cette extrémité.

2. Ensemble selon la revendication 1, dans lequel l'élément de liaison (32) assure une liaison permanente effective entre l'extrémité distale de l'élément poussoir et le cathéter à fil de guidage.

3. Ensemble selon la revendication 1, dans lequel l'élément de liaison (32) peut être rompu pour permettre un déplacement relatif entre le cathéter à fil de guidage et l'élément poussoir.

4. Ensemble selon la revendication 1, dans lequel l'élément de liaison (32) est une colle durcissable aux ultraviolets.

5. Ensemble selon la revendication 1, dans lequel l'élément de liaison (32) est une colle au cyanoacrylate.

6. Ensemble selon la revendication 1, dans lequel l'élément de liaison (32) est une partie fondue de l'élément poussoir ou du cathéter à fil de guidage.

7. Procédé de fabrication d'un ensemble d'introduction (10), le procédé comportant les étapes qui consistent à :
prévoir un cathéter (12) à fil de guidage,
ajuster concentriquement un élément poussoir allongé (16) sur le cathéter (12) à fil de guidage, le cathéter (12) à fil de guidage et l'élément poussoir (16) présentant des extrémités distales respectives (20, 18) et des extrémités proximales respectives (24, 26),
raccorder une unité externe de manipulation (28) aux extrémités proximales (24, 26) de l'élément poussoir (16) et du cathéter (12) à fil de guidage et
prévoir un dispositif de prévention de la rotation entre l'élément poussoir (16) et le cathéter (12) à fil de guidage, sur l'extrémité distale (18) de l'élément poussoir (16) ou à proximité de cette dernière, de manière à relier l'extrémité distale (18) de l'élément poussoir (16) au cathéter (12) à fil de guidage,
l'étape consistant à prévoir un dispositif de prévention de la rotation comprenant l'étape consistant à appliquer entre l'élément poussoir (16) et le cathéter (12) à fil de guidage un élément de liaison (32), à l'extrémité distale (18) de l'élément poussoir (16) ou à proximité de cette dernière.

8. Procédé selon la revendication 7, dans lequel l'élément de liaison (32) assure une liaison permanente effective entre l'extrémité distale (18) de l'élément poussoir (16) et le cathéter à fil de guidage.

9. Procédé selon la revendication 7, dans lequel l'élément de liaison (32) peut être rompu pour permettre un déplacement relatif entre le cathéter (12) à fil de guidage et l'élément poussoir (16).

10. Procédé selon la revendication 7, dans lequel l'élément de liaison (32) est une colle durcissable aux ultraviolets.

11. Procédé selon la revendication 7, dans lequel l'élément de liaison (32) est une colle au cyanoacrylate.

12. Procédé selon la revendication 7, dans lequel l'élément de liaison (32) est une partie fondue de l'élément poussoir (16) ou du cathéter (12) à fil de guidage, le procédé comportant l'étape qui consiste à faire fondre une partie de l'élément poussoir (16) et/ou du cathéter (12) à fil de guidage pour réaliser ledit élément de liaison (32).
